# EUROPEAN PATENT APPLICATION

(11) **EP 3 378 451 A1**
(43) Date of publication of application: **26.09.2018**
(21) Application number: 17382149.7
(22) Date of filing: 24.03.2017
(51) Int. Cl.: A61F 13/02, A61F 13/00

(54) **A SANITARY DRESSING**

(71) Applicant: Iberhospitex S.A., 08185 Lliça de Vall (Barcelona) (ES)
(72) Inventor: LÓPEZ MOYA, Mario, 08185 LLIÇÀ DE VALL (ES); COLL PÉREZ, Marc, 08185 LLIÇÀ DE VALL (ES); MUSTETI, Ana Daniela, 08185 LLIÇÀ DE VALL (ES); GAMBUS MILLET, Montserrat, 08185 LLIÇÀ DE VALL (ES)
(74) Representative: ZBM Patents - Zea, Barlocci & Markvardsen

(57) **Abstract**

A sanitary dressing for protecting a protruding portion of a catheter implanted into a patient body through a skin entry site is provided. The sanitary dressing comprises a sheath for receiving the catheter. The sheath comprises a first opening enabling the portion of the catheter protruding from a skin entry site to be received in the sheath, and a second opening fitted with a closing mechanism for gaining access to the protruding portion of the catheter. The sanitary dressing further comprises an adhesive layer for adhering the sanitary dressing to the patient skin.

## Description

The present disclosure relates to sanitary dressings, more specifically to sanitary dressings for protecting a protruding portion of a catheter inserted into a patient body.

### BACKGROUND

During the medical care of patients catheters may be used to access the circulatory system for the purposes of blood extraction, hydration of the patient, administering of medication and kidney dialysis, among others.

A catheter may typically include an insertion end that penetrates into the patient's circulatory system through an orifice in the skin, a valve mechanism that closes the access to the vein when the device is not in use, and a connection port that allows the connection of an external device such as an IV (intravenous) drip source, syringe, etc. Such a catheter may remain in the body for an extended period of time, from days to months, or even years, such that there is an unhealed open orifice, i.e. the skin entry site, from which a portion of the catheter protrudes. In order to protect the exposed portion of the catheter and prevent infections at the catheter entry site, it is known to cover the wound with a self-adhesive sanitary dressing.

However, to gain access to the catheter, e.g. each time a drug is administered, known sanitary dressings need to be removed from the patient skin. Moreover, once the dressing is removed it is no longer usable e.g. because the adhesive layer is damaged or due to the textile is creased, and thus, it needs to be replaced by a new dressing. This involves frequent exposure of the catheter entry site and therefore a higher risk of infection. Additionally, as several dressings are used during the treatment, the overall costs related to sanitary supplies increase.

In conclusion, it may be convenient to provide a sanitary dressing that may be maintained adhered to the skin for a longer time, reducing the frequency of dressing removal and substitution, and which at least partially solves some of the aforementioned problems.

### SUMMARY

In a first aspect, a sanitary dressing for protecting a protruding portion of a catheter implanted into a patient body through a skin entry site is provided. The sanitary dressing comprises a sheath for receiving the catheter. The sheath further comprises a first opening and a second opening. The first opening enables the portion of the catheter protruding from a skin entry site to be received in the sheath. The second opening is fitted with a closing mechanism to open/close the second opening for gaining access to the protruding portion of the catheter. The sanitary dressing further comprises an adhesive layer for adhering the sanitary dressing to the patient skin.

The provision of a second opening having a closing mechanism enables gaining access to the catheter, for example for treatment, without removing the dressing, thus the skin entry site is not frequently exposed and the risk of infection is reduced.

Additionally, as there is no need to discard a sanitary dressing each time the catheter needs to be accessed e.g. each time a drug is administered the amount of sanitary dressings to be used during the treatment substantially decreases (which also reduces the costs of sanitary supplies). Furthermore, the unpleasant removing of an adhesive adhered to the skin does not need to be frequently repeated and therefore, the patient comfort is improved.

In some examples, the dressing may comprise an exterior and an interior textile sheets which may be joined to form the sheath and may comprise an outer hydrophobic surface, preferably the outer hydrophobic surface may be a superhydrophobic surface. Using a superhydrophobic outer surface prevents the dressing from being prematurely damaged during such long-term use, even in case it is repeatedly soaked in water, for example if the patient showers regularly.

The exterior and interior sheets may, for example, be woven or non-woven textile sheets.

In some examples, the exterior and interior sheets may be joined such as to form the second opening at or near one end of the sheath opposite, i.e. remote, from the first opening, or on one side of the sheath between the end of the sheath with the first opening and the opposite end of the sheath.

In some examples, the closing mechanism may be selected for example among a re-closable zip, a re-closable zip with a slider or a hook-and-loop fastener.

In some examples, the sanitary dressing further comprises an adhesive sheet comprising the adhesive layer, a polyurethane layer and a non-woven textile layer arranged between the adhesive layer and the polyurethane layer.

In some examples the polyurethane layer may be transparent, and a transparent zone may be formed in the adhesive sheet: in some examples the transparent zone may be formed by a cut-out in the non-woven textile layer, such that in this zone the adhesive sheet only comprises the transparent polyurethane layer.

In some examples of the sanitary dressing there may be an overlap between the adhesive sheet and the interior sheet, and the interior sheet may comprise adhesive at least in the portion of overlap with the adhesive sheet, such that the dressing may be adhered to the skin of the patient all around the skin entry site. With this solution the entry site is completely isolated from the environment, and this affords an increased protection against infection.

Such a transparent zone may be for example in correspondence with the skin entry site so as to enable frequent inspection of the site, assess the condition of the orifice in the skin, and prevent infection.

It may be an advantage in some examples that the zone of the adhesive sheet intended to overlap the skin entry site, which may coincide at least partly with the transparent zone, has a high Moisture Vapour Transmission Rate (MVTR), so that exudates may evaporate easily.

In some examples, the sanitary dressing comprises a transdermal patch placed in correspondence with the skin entry site, for example attached to the adhesive sheet, to release medicaments at the skin entry site and reducing the risk of infection.

### BRIEF DESCRIPTION OF THE DRAWINGS

Non-limiting examples of the present disclosure will be described in the following, with reference to the appended drawings, in which:
Figure 1 schematically illustrates a top view of a sanitary dressing according to an example;
Figure 2 schematically illustrates a lateral view of a sanitary dressing according to an example; and
Figure 3 schematically illustrates a top view of a sanitary dressing according to an example.

### DETAILED DESCRIPTION

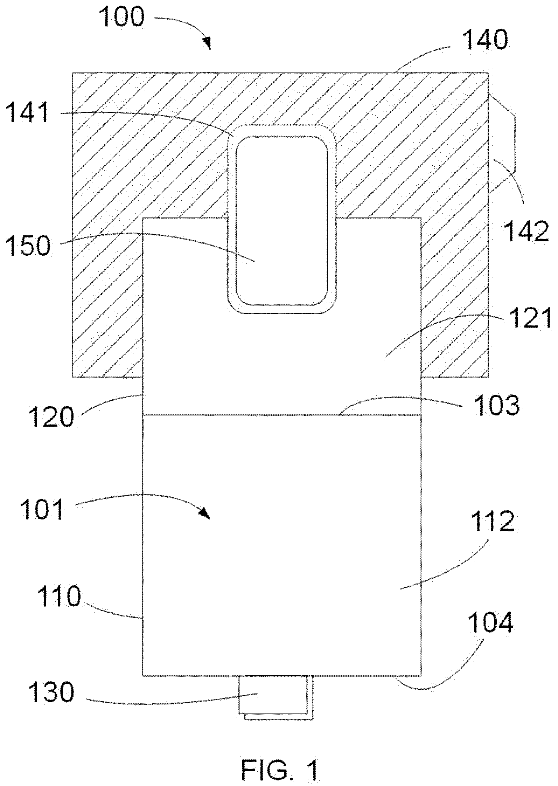

Figure 1 shows a sanitary dressing 100 as seen from the side intended to contact the patient skin. It comprises a sheath 101 to receive the protruding portion of a catheter inserted into a patient body at a skin entry site. The sheath 101 may be formed by an interior sheet 110 and an exterior sheet 120 joined together at the edges e.g. by adhesive, stitching, or other.

The size and/or shape of the sheath 101 (and of both interior and exterior textile sheets) may depend on the catheter type to be housed and/or the position in which the dressing is to be adhered.

The interior and exterior sheets 110, 120 may be made of e.g. polyethylene terephthalate (PET), polyolefin, woven material(s), non-woven material(s), breathable material(s) and/or material(s) capable of being wrinkled i.e. to easily gain access to the catheter, but stiff enough to form a protective sheath.

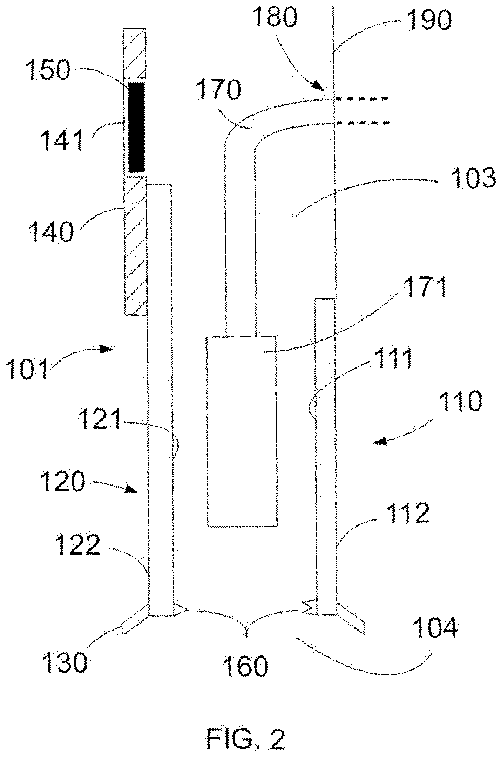

The interior and exterior sheets 110, 120 may comprise inner surfaces 111 (see Figure 2), 121 and outer surfaces 112, 122 (see Figure 2). The outer surfaces 112, 122 may be hydrophobic or preferably superhydrophobic to protect the dressing from moisture e.g. when the patient takes a shower, thus forming an impermeable sheath.

A superhydrophobic (or ultrahydrophobic) surface is defined herein as that in which the contact angle of a water drop on the surface exceeds 150°.

Besides, at least one of the inner surfaces 111, 121 may be hydrophilic. A hydrophilic surface absorbs water or water-based compounds e.g. blood or any corporal fluid secreted from the skin entry site, and therefore it is used to maintain the inner portion of the sheath dry.

The interior and exterior sheets 110, 120 may be overlapped and joined together so as to create a first opening 103 which enables the catheter protruding portion to enter into the sheath 101 and be housed therein. The first opening 103 may be located at one end of the sheath 101 wherein the exterior sheet 120 extends beyond the interior sheet 110.

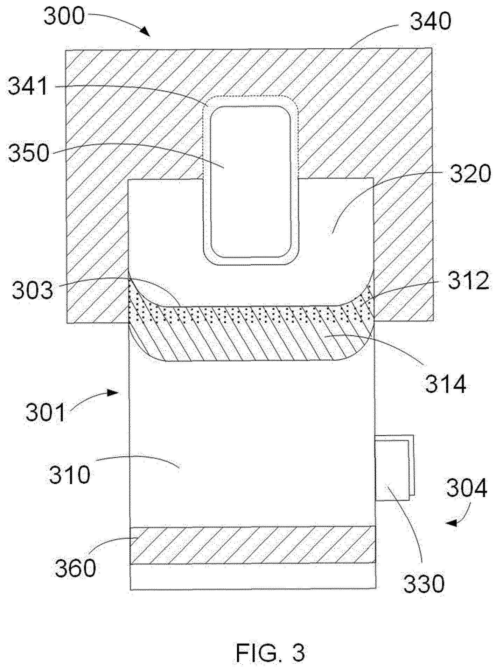

The interior and exterior sheets 110, 120 may also be joined in such a way to create a second opening 104 which may comprise a releasable closing mechanism (disclosed later on) through which e.g. the sanitary staff may easily gain access to the protruding portion of the catheter. The second opening 104 may be placed opposite the first opening as shown in Figure 1. In other examples (see Figure 3), the second opening may be placed on one side of the sheath, between the end with the first opening and the opposite end of the sheath 101. As shown in Figure 1, tabs 130 may be added to facilitate opening/closing of such closing mechanism.

The sanitary dressing 100 may further comprise an adhesive sheet 140 to adhere the sanitary dressing 100 to the patient skin around the skin entry site. The adhesive sheet 140 may comprise an adhesive layer, a non-woven textile layer for structural resistance and/or support, and a polyurethane layer, which may give structural support, may be hydrophobic and/or may act as an antibacterial barrier. The polyurethane layer may also be transparent. The exposed side of the polyurethane layer may have a superhydrophobic surface.

In some examples, the sanitary dressing may have an adhesive layer on the exterior sheet instead of having a separate adhesive sheet 140.

In order to be able to monitor the skin entry site, the adhesive sheet 140 may comprise a transparent zone 141 in correspondence with the skin entry site (see Figure 2), that is, a zone which enables visual inspection of the orifice from which the catheter projects and of the skin condition without removing the dressing.

Since the non-woven layer may be opaque, this non-woven layer and the adhesive layer attached to it may be absent in the transparent zone: for example an opening may be cut out in the non-woven layer, such that in the transparent zone the adhesive sheet may comprise just the transparent polyurethane layer.

In some examples, the dressing may comprise a release sheet (not fully shown) to protect the adhesive layer. In some examples, in order to facilitate its removal the release sheet may be split in two different sheets, and/or it may comprise a tab 142 that projects with respect to the adhesive sheet 140.

In some examples (not shown), the sanitary dressing may comprise a further release sheet to protect also the outer surface of the polyurethane layer until the dressing has been applied to the patient. Such release layer may comprise at least one outwardly protruding tab to aseptically attach the adhesive dressing to patient skin.

Figure 1 further shows a transdermal patch 150 that may be placed in correspondence with the skin entry site with the purpose of releasing a medicament while the dressing is attached to the patient skin. The patch 150 may comprise a hydrocolloid with an antiseptic agent e.g. to avoid infections. Several antiseptic agents may be used e.g. Chlorhexidine dihydrochloride, Chlorhexidine digluconate, Silver (ions, nanoparticles), Iodine, polyhexamethylene biguanide, etc. The patch 150 may be attached to the adhesive sheet in the transparent zone thereof in correspondence with the opening in the non-woven layer, for example adhered to the inner side of the polyurethane layer. It may be made of a relatively transparent or translucent material to allow the observation of the skin entry site.

Figure 2 depicts, in irregular proportions for the sake of clarity, a very schematic section view of the sanitary dressing 100 of Figure 1 once adhered onto the patient skin 190. The figure represents only the cut along a plane perpendicular to the plane of the dressing, and therefore the sides along which the exterior and interior sheets are stitched or otherwise joined together are not shown.

In the figure, the two parts of the closing mechanism 160 are shown detached (in the "open" position), enabling the sanitary staff to reach the protruding portion of the catheter through the second opening 104. Figure 2 also shows the protruding portion of catheter 170 passing through the first opening 103 to be housed within the sheath 101.

Additionally, Figure 2 shows how the catheter 170 protrudes from an orifice 180 made in the patient skin 190. The catheter in this example comprises a connection port 171 in which several accessories e.g. optical module, fluid injector, etc., may be connected. In other examples different catheter types may be used.

Figure 2 shows the sheath 101 in which the protruding portion of the catheter 170 is received. The exterior and interior sheets 110, 120 which form the sheath 101 are also shown in Figure 2. The inner surfaces 111, 121 and outer surfaces 112, 122 of both interior and exterior layers are also depicted.

The closing mechanism 160, comprising a pair of complementary mating portions is also shown in Figure 2. In this example, the closing mechanism 160 is a re-closable zip. In other examples, the re-closable zip may comprise a slider to facilitate the open/close of the second opening. In further examples, a hook-and-loop mechanism may be used as closing mechanism. Additionally, a pair of tabs 130 may be added to interior and exterior sheets 110, 120 so as to facilitate the opening/closing of the closing mechanism 160.

Figure 2 further depicts an adhesive sheet 140 comprising a transparent zone 141. The adhesive sheet 140 may comprise a polyurethane layer, a non-woven textile layer and an adhesive layer (not referenced), except for the zone in correspondence with the skin entry site, i.e. the transparent zone 141, which may only comprise a polyurethane layer so as to observe the orifice.

A transdermal patch 150 as described above is shown in Figure 2 attached to the adhesive layer 140 in the transparent 141.

The part of the sanitary dressing overlapping or in correspondence with the skin entry site, i.e. the zone that may be transparent, may also be breathable to allow evaporating the moisture caused by e.g. sweat, blood, etc., around the orifice. Therefore the material(s) of the dressing in correspondence with the skin entry site, and particularly those of the adhesive sheet in this zone, may preferably have an elevated Moisture Vapour Transmission Rate (MVTR). By an elevated MVTR it is meant a value that allows enough evaporation to maintain healthy conditions at the skin entry site during prolonged use of the dressing. In an example, MVTR may be greater than 600 g/(m²x24h), that is, 600 g per square meter per day.

The adhesive sheet may have several layers, as explained above. The layer of polyurethane may be very thin and its MVTR may therefore be quite high; however, the transdermal patch placed in this zone may be thicker and the MVTR of the material may be too low: in some examples a plurality of vents, such as perforations (not shown), may be made in the patch to increase the overall MVTR of the patch and enable the moisture drops to pass through it.

Figure 3 shows another example of a sanitary dressing 300 as disclosed herein. Sanitary dressing 300 comprises a sheath 301, an adhesive sheet 340 that may comprise a transparent zone 341, and may comprise a transdermal patch 350, as disclosed above.

In the sanitary dressing 300 of Figure 3 the second opening 304 for accessing the catheter, e.g. to deliver some treatment to the patient, may be placed on one side of the sheath 301, between the end of the sheath where the first opening 303 may be arranged and the opposite end of the sheath.

Figure 3 also shows tabs 330 which may facilitate the opening/closing of the closing mechanism (not shown). The closing mechanism and other features of the dressing may be as in the previous example.

The interior layer may, as in the example shown in Figure 3, partly overlap the adhesive sheet 340: the portion of the interior layer 310 that overlaps with the adhesive sheet 340 has been indicated in Figure 3 by dotting, and with reference numeral 312. It is understood that this "overlapping" is merely geometrical, since the exterior layer 320 extends between the interior layer 310 and the adhesive sheet 340.

An adhesive 314 may be added on the outer surface of the interior sheet 310 at least in the part that overlaps the adhesive layer 340: this adhesive 314 will also attach the dressing to the skin of the patient, and may be covered by the same release sheet provided for the adhesive sheet 340.

It will be understood that in this case, by virtue of the overlap and the provision of adhesive 314, a continuous adhesive area completely enclosing the skin entry site is formed, and therefore the skin entry site may be better protected from the environment.

Such a continuous adhesive area completely enclosing the skin entry site such as described in relation with the implementation of Figure 3 may also be provided in implementations such as those of Figures 1 or 2.

In some examples, the edge of the interior sheet 310 overlapping portion of the adhesive sheet 340 may comprise a predetermined shape e.g. straight, substantially curved.

The example of Figure 3 may further comprise a further adhesive 360 which may be placed in the interior textile sheet. Such adhesive sheet may be used to increase the attachment of the dressing to patient skin.

In some examples, the adhesive sheet 140, 340 and/or adhesives 314, 360 may be repositionable and/or non-traumatic adhesives to facilitate the attachment/detachment of the sanitary dressing.

Sanitary dressings such as disclosed herein may be attached to the skin of a patient after a catheter has been implanted at a skin entry site, and maintained in place for several days or for a few weeks without becoming damaged or deteriorated even if is frequently splashed with water such as in a shower. At the same time medical practitioners may both monitor the health condition of the entry site through the transparent zone and access the catheter for treatment through the second opening.

To this end, the closing mechanism may be manually opened, e.g. in a safe medical environment, and the sheath may be slightly wrinkled up to easily connect the desired accessory to the catheter. After treatment the accessory is disconnected, the sheath extended again over the catheter, and closed by the closing mechanism, so that the catheter is protected from the environment.

Although only a number of examples have been disclosed herein, other alternatives, modifications, uses and/or equivalents thereof are possible. Furthermore, all possible combinations of the described examples are also covered. Thus, the scope of the present disclosure should not be limited by particular examples, but should be determined only by a fair reading of the claims that follow. If reference signs related to drawings are placed in parentheses in a claim, they are solely for attempting to increase the intelligibility of the claim, and shall not be construed as limiting the scope of the claim.

## Claims

1. A sanitary dressing for protecting a protruding portion of a catheter implanted into a patient body through a skin entry site, the sanitary dressing comprising a sheath for receiving the catheter, and wherein the sheath comprises a first opening for enabling the portion of the catheter protruding from the skin entry site to be received in the sheath, and a second opening fitted with a closing mechanism to open/close the second opening for gaining access to the protruding portion of the catheter, the sanitary dressing further comprising an adhesive layer for adhering the sanitary dressing to the patient skin.

2. The sanitary dressing according to claim 1, wherein the sanitary dressing comprises an exterior and an interior textile sheets joined to form the sheath, the textile sheets comprising an outer hydrophobic surface, preferably a superhydrophobic surface.

3. The sanitary dressing according to claim 2, wherein the exterior sheet extends beyond the interior sheet at one end of the sheath and the first opening is defined at this end of the sheath.

4. The sanitary dressing according to claim 3, wherein the exterior and interior sheets are joined such as to form the second opening at or near an end of the sheath opposite to the first opening.

5. The sanitary dressing according to claim 3, wherein the exterior and interior sheets are joined such as to form the second opening on one side of the sheath, between the end of the sheath with the first opening and the opposite end of the sheath.

6. The sanitary dressing according to any of claims 1 to 5, wherein the closing mechanism of the second opening is selected among a re-closable zip, a reclosable zip with a slider or a hook-and-loop fastener.

7. The sanitary dressing according to any of claims 1 to 6, further comprising an adhesive sheet comprising the adhesive layer, a polyurethane layer and a non-woven textile layer arranged between the adhesive layer and the polyurethane layer.

8. The sanitary dressing according to claim 7, wherein there is an overlap between the adhesive sheet and the interior sheet.

9. The sanitary dressing according to claim 8 wherein an outer surface of the interior sheet comprises an adhesive layer at least in the portion of overlap with the adhesive sheet, whereby the sanitary dressing may be adhered to the skin all around the skin entry site.

10. The sanitary dressing according to any of claims 7 to 9, wherein the adhesive sheet comprises a transparent zone intended to overlap the skin entry site.

11. The sanitary dressing according to claim 10, wherein the adhesive sheet comprises a non-woven textile layer having a cut-out to form the transparent zone of the adhesive sheet.

12. The sanitary dressing according to any of claims 7 to 11, wherein the zone of the adhesive sheet intended to overlap the skin entry site has a high Moisture Vapour Transmission Rate (MVTR).

13. The sanitary dressing according to any of claims 7 to 12, further comprising a transdermal patch attached to the adhesive sheet in the zone intended to overlap the skin entry site.

14. The sanitary dressing according to any of claims 2 - 13, further comprising an adhesive layer on an outer surface of the interior sheet at the end of the interior sheet remote from the first opening.

15. The sanitary dressing according to claim 14, wherein the adhesive layer and/or adhesive sheet may be repositionable and/or non-traumatic adhesive to facilitate the attachment/detachment of the sanitary dressing.
